Europäisches Patentamt

European Patent Office

Office européen des brevets

(19)

(11) **EP 1 158 288 A1**

(12) ## DEMANDE DE BREVET EUROPEEN

(43) Date de publication:
**28.11.2001 Bulletin 2001/48**

(51) Int Cl.⁷: $G01N\ 1/28$, $G01M\ 11/00$, $G01N\ 33/00$

(21) Numéro de dépôt: **00111371.1**

(22) Date de dépôt: **26.05.2000**

(84) Etats contractants désignés:
**AT BE CH CY DE DK ES FI FR GB GR IE IT LI LU MC NL PT SE**
Etats d'extension désignés:
**AL LT LV MK RO SI**

(71) Demandeur: **Technodop Ltd. (Société de Droit Irlandais)**
**Dublin 2 (IE)**

(72) Inventeurs:
• **Cohen-Adad, Marie-Thérèse**
**69300 Caluire (FR)**

• **Laversenne, Leatitia**
**69007 Lyon (FR)**
• **Goutaudier, Christelle**
**69250 Fleurieu/Saone (FR)**
• **Boulon, Georges**
**69005 Lyon (FR)**

(74) Mandataire: **Gaucherand, Michel**
**IXAS Conseil**
**15 Rue Emile Zola**
**69002 Lyon (FR)**

(54) **Procéde d'optimisation rapide des caractéristiques physiques de matériaux solides, pouvant être des métaux ou des composés chimiques, par détermination de la composition idéale pour lesdits matériaux**

(57) L'invention concerne un procédé d'optimisation d'au moins une caractéristique physique souhaitée pour un matériau formé d'au moins deux constituants, qui peuvent être des métaux ou des composés chimiques solides, rendus miscibles par fusion de zone, par détermination de la composition idéale dudit matériau conduisant à la valeur optimale de ladite caractéristique, ainsi que les matériaux optimisés de composition idéale résultant des étapes de préparation et d'analyses du procédé et l'application de ces matériaux.

EP 1 158 288 A1

## Description

Domaine de l'invention

La présente invention concerne un procédé d'optimisation d'au moins une caractéristique physique souhaitée pour un matériau solide formé à partir d'au moins deux constituants, qui peuvent être des métaux ou des composés chimiques solides, rendus miscibles par fusion de zone, par détermination de la composition idéale dudit matériau conduisant à la valeur optimale de ladite caractéristique.

Il convient de noter que l'optimisation d'une caractéristique physique n'est pas forcément la détermination de sa valeur maximale, mais la détermination de la valeur la plus appropriée souhaitée (c'est-à-dire optimale) de ladite caractéristique pour un matériau donné selon l'utilisation envisagée de ce matériau.

Par conséquent, suivant le domaine d'application envisagé, la valeur la plus appropriée (c'est-à-dire optimale) de la caractéristique physique ne sera pas obligatoirement la valeur maximale de ladite caractéristique. Par exemple, dans le domaine des transferts thermiques, pour un matériau utilisé comme isolant thermique, la valeur optimale de la conductibilité thermique souhaitée sera la valeur de la conductibilité thermique minimum, alors que pour un matériau laser, on recherchera la conductibilité thermique la plus élevée en raison de l'obligation de dissiper très rapidement la chaleur créée au sein du matériau par l'irradiation excitatrice.

Ainsi, le procédé selon l'invention permet de déterminer l'évolution d'au moins une caractéristique physique d'un matériau en fonction de la composition dudit matériau, et en outre permet de définir le domaine de composition idéale pour ledit matériau conduisant à la valeur optimale de ladite caractéristique en fonction de l'application recherchée.

L'invention concerne plus particulièrement un procédé d'optimisation d'au moins une caractéristique physique souhaitée pour un matériau formé à partir d'au moins deux constituants, qui peuvent être des métaux ou des composés chimiques solides, qui sont rendus miscibles par fusion de zone, procédé comprenant la mesure, non destructive et effectuée point par point, de la composition et d'au moins une caractéristique physique possédée par le dit matériau, puis comprenant la détermination de la composition idéale correspondante à la valeur optimale de la caractéristique physique souhaitée.

L'invention concerne également les matériaux optimisés de composition idéale résultant des étapes de préparation et d'analyses du procédé, et comportant la caractéristique physique optimale souhaitée.

L'invention concerne par ailleurs la réalisation du matériau, de forme parallélépipédique ou cylindrique, formé à partir d'au moins deux constituants rendus miscibles par fusion de zone, intervenant dans le procédé de l'invention.

L'invention concerne enfin l'application des matériaux optimisés de composition idéale résultant du procédé de l'invention et comportant la caractéristique physique souhaitée rendue optimale.

Etat de la technique

Plusieurs méthodes destinées à améliorer la formulation de matériaux complexes, notamment destinées à optimiser une, voire les propriété(s) physique(s) générée(s) par le matériau concerné en fonction de la composition de celui-ci, ont été envisagées. En effet, l'utilisation de l'approche combinatoire dans le but d'optimiser des matériaux, formés d'au moins deux constituants, présentant des propriétés de luminescence a fait l'objet de nombreuses publications [voir, par exemple, Xiao-Dong Sun, Chen Gao, Jingsong Wang, and X.-D. Xiang, Appl. Phys. Lett., 70(25), 3353 (1997) ou encore Earl Danielson, Josh H. Golden, Eric W. McFarland, Casper M. Reaves, W.Henry Weinberg & Xin Di Wu, Nature, 389, 944(1997)].

L'une de ces publications parue dans le journal Nature et référencée ci-dessus décrit la réalisation de matériauthèques (matrices de compositions comportant au moins deux constituants) dont les matériaux présentent des propriétés de luminescence. Après traitement des matériauthèques à des températures variables, on définit par des analyses quelle zone de ces matériauthèques présente la plus favorable chromaticité. Ainsi pour déterminer dans la zone de matériauthèques sélectionnée quelle est la composition idéale du matériau pour la chromaticité désirée, une seconde matériauthèque est synthétisée. Par cette approche, une nouvelle composition quantitative du matériau en question présentant une chromaticité «optimisée» a pu être identifiée. Cette méthode par approximation apparaît être longue de part le fait qu'elle nécessite plusieurs étapes, notamment une étape consistant en l'établissement de mélanges variés de constituants suivie d'une étape d'affinement consistant en l'établissement de mélanges sélectionnés présentant la caractéristique souhaitée. Après sélection du mélange présentant la propriété optimale, un échantillon dudit mélange est préparé par les voies conventionnelles de la synthèse chimique, puis sur cet échantillon seront effectuées les différentes analyses chimiques. De plus, cette méthode s'avère lourde en nombre d'expérimentations et ne concerne que l'élaboration de poudres.

Une autre méthode, qui est celle des plans d'expérience, nécessite moins de points expérimentaux que la méthode précédemment citée mais elle ne permet pas d'avoir connaissance de la composition du matériau mesurée point par point. En effet, selon cette méthode, utilisée par exemple afin de déterminer la composition d'un matériau pour une propriété physique choisie et en fonction du traitement thermique fixé, des échantillons de compositions différentes sont préparés et soumis au traitement thermique choisi. L'utilisation de plans d'expérience va permettre de délimiter ou tout du moins de cibler une zone pour laquelle les compositions qui, une fois soumises au protocole de cuisson, présenteront les critères recherchés pour la formulation du matériau. Malgré un traitement informatique des résultats, la mise au point d'une formulation selon la méthode de plans d'expérience peut parfois aboutir à la préparation de très nombreux échantillons. De plus, cette méthode utilisée notamment pour l'optimisation de matériaux binaires devient très laborieuse lors de la formulation de matériaux plus complexes.

Par ailleurs, l'une et l'autre des méthodes précédemment évoquées c'est-à-dire l'approche combinatoire et la méthode de plans d'expérience nécessitent la réalisation d'échantillons propres à chaque mesure. Il s'agit donc de méthodes de mesures discontinues, qui présentent une certaine lourdeur due au nombre d'expérimentations nécessaires pour déterminer la composition permettant l'optimisation de la propriété souhaitée.

Une autre publication [voir M. Th. Cohen-Adad, M. Gharbi, C. Goutaudier, R. Cohen-Adad, Journal of Alloys and Compounds, 289, (1999) 185-196] décrit quant à elle une approche combinatoire dans le domaine des alliages métalliques, méthode basée sur la rapide détermination de diagrammes de phase de ces alliages en utilisant la technique de la fusion de zone. Cet article décrit l'utilisation de la technique précédemment citée pratiquée sur un barreau hétérogène, constitué de deux métaux ou alliages A et B, dans le but d'étudier les équilibres liquide-solide d'un système binaire (alliage Al-Co). En fait, le barreau obtenu après fusion de zone est polyphasé et l'observation des phases est réalisée par une analyse in situ sur des minces bandes découpées à différentes distances le long du barreau. Cette méthode permet de suivre l'évolution des différentes phases et d'établir le spectre des phases solides et des transformations de phases dans un diagramme de phases. Les auteurs mentionnent en outre qu'un diagramme de phases associé à des propriétés mesurées in situ le long du barreau est un bon moyen en vue de la recherche de matériaux optimisés. Cette méthode n'a pour but que l'obtention de la composition des phases obtenues au cours du refroidissement d'un liquide, le barreau obtenu après fusion de zone étant polyphasé.

Par rapport à l'état de la technique de l'art antérieur mentionné précédemment, qui décrit des mesures discontinues, l'invention s'applique à des matériaux monophasés et permet une continuité de la mesure envisagée (à savoir une mesure point par point de la composition et/ou d'au moins une caractéristique physique souhaitée, ces mesures étant non destructives).

Malgré l'abondance de travaux réalisés dans ce domaine, l'activité de recherche reste soutenue, notamment dans le but de trouver des matériaux présentant une ou des propriétés physiques optimisées et dont la composition soit améliorée.

Par la suite, pour faciliter la lecture du texte exposant l'invention, on appellera «barreau» tous les matériaux de l'invention élaborés sous forme parallélépipédique ou cylindrique et obtenus avant la fusion de zone (fusion effectuée selon l'une des techniques de fusion de zone).

De même, par la suite, on appellera «fibre» tous les matériaux de l'invention issus du barreau et élaborés après la fusion de zone.

Objet de l'invention

La présente invention apporte une contribution nouvelle et surprenante dans ce domaine. En effet, le but de la présente invention est de créer une méthode ne nécessitant l'utilisation que d'un très petit nombre d'échantillons, se présentant sous la forme d'une fibre monophasée monocristalline ou polycristalline ou vitreuse, élaborés selon une technique de fusion de zone à partir d'un matériau formé d'au moins deux constituants, qui peuvent être des métaux ou des composés chimiques solides, mis en oeuvre pour créer un barreau.

Un objet de l'invention est de pouvoir faire des mesures point par point de la composition d'un matériau, à savoir une fibre monophasée monocristalline ou polycristalline ou vitreuse à gradient de composition telle que définie ci-dessus, sans destruction du matériau analysé.

Un autre objet de l'invention est d'effectuer sur le même matériau des mesures, non destructives, d'au moins une caractéristique physique souhaitée, et ceci point par point. Il s'agit par conséquent d'une méthode permettant d'associer des mesures de composition avec des mesures de caractéristiques physiques souhaitées pour un même matériau.

Un autre objet de l'invention est de pouvoir déterminer la composition idéale correspondante à la valeur optimale de la ou des caractéristiques physiques souhaitées pour un matériau donné sans destruction dudit matériau.

Un autre objet de la présente invention est les matériaux optimisés (tels que, par exemple, les matériaux laser, les fibres optiques) de composition idéale résultant des étapes de préparation et d'analyses du procédé, et comportant la caractéristique physique optimale souhaitée.

Sommaire de l'invention

La présente invention est basée sur l'observation qu'un procédé permettant l'optimisation d'au moins une caractéristique physique souhaitée pour un matériau défini selon l'invention, par, détermination de la composition idéale dudit matériau conduisant à la valeur optimale de ladite caractéristique pouvait éviter toute une série d'expérimentations complexes de façon efficace.

Ainsi, l'invention a pour objet un procédé d'optimisation d'au moins une caractéristique physique souhaitée pour un matériau formé à partir d'au moins deux constituants, qui peuvent être des métaux ou des composés chimiques solides, rendus miscibles par fusion de zone, par détermination de la composition idéale ($C_I$) dudit matériau conduisant à la valeur optimale ($R_I$) de ladite caractéristique, ce procédé étant caractérisé par le fait qu'il comporte les étapes suivantes :

(a) la préparation d'une fibre monophasée monocristalline ou polycristalline ou vitreuse à gradient de composition (FG) continu, à partir d'un matériau formé d'au moins deux constituants, qui peuvent être des métaux ou des composés chimiques solides, élaborée selon une technique de fusion de zone;

(b) la détermination, sans destruction de la fibre, de la composition ($C_{FG}$) par une mesure point par point tout au long de ladite fibre et l'établissement de la courbe de composition correspondante;

(c) la détermination sur la même fibre de la réponse physique ($R_{FG}$) d'au moins une caractéristique physique souhaitée par une mesure point par point, non destructive, et l'établissement de la courbe de la réponse physique correspondante;

(d) la préparation d'une fibre de référence ($F_R$) de composition homogène ($C_{FR}$), mettant en oeuvre les constituants, sur laquelle on mesure exactement en au moins un point la composition ($C_{FR}$) et la réponse physique ($R_{FR}$) correspondante à au moins une caractéristique physique souhaitée pour permettre l'établissement de la corrélation entre la composition ($C_{FG}$) et la réponse physique ($R_{FG}$) correspondante à au moins une caractéristique physique souhaitée mesurées sur la fibre ($F_G$) ;

(e) la mise en correspondance pour la fibre ($F_G$) de la courbe de composition ($C_{FG}$) et de la courbe de la réponse physique ($R_{FG}$) d'au moins une caractéristique physique souhaitée par la mise en concordance, sur une même verticale des valeurs ($C_{FR}$) et ($R_{FR}$) reportées sur les courbes respectives de la fibre $F_G$;

(f) l'optimisation de la composition. d'une fibre par lecture directe, sur la courbe de composition de la fibre ($F_G$), de la composition idéale ($C_I$) correspondant à la valeur optimale ($R_I$) de la réponse physique de la caractéristique physique souhaitée.

Description détaillée de l'invention

Le matériau permettant l'élaboration d'une fibre monophasée monocristalline ou polycristalline ou vitreuse à gradient de composition intervenant dans l'étape a) du procédé de l'invention, est tout d'abord réalisé. Ce matériau est formé à partir d'au moins deux constituants qui peuvent être des métaux ou des composés chimiques solides. A titre de matériaux formés d'au moins deux constituants, par exemple, d'origine métallique et/ou minérale, on peut envisager des mélanges de métaux, d'oxydes métalliques ou autres matières minérales. On peut citer par exemple les alliages métalliques, toutes les céramiques industrielles et en particulier les céramiques hautement réfractaires, ou les matériaux minéraux binaires ou d'ordre supérieur. On peut mentionner à titre d'exemples, les alliages aluminium-cobalt, fer-cobalt, fer-aluminium ou les céramiques réfractaires à base d'oxyde de gadolinium et d'oxyde d'yttrium ou les céramiques utilisées dans la fabrication des porcelaines.

Ce matériau, permettant l'élaboration de la fibre monophasée monocristalline ou polycristalline ou vitreuse à gradient de composition, se présente sous la forme d'un barreau hétérogène en composition formé à partir d'au moins deux constituants tels que définis précédemment.

Ces barreaux hétérogènes ont généralement les dimensions usuelles de l'art. Des dimensions de l'ordre de 1 centimètre à plus d'un mètre de longueur et de l'ordre de 0,5 à 3 centimètres de diamètre sont, par exemple, considérer comme des dimensions usuelles pour réaliser un barreau tel que défini précédemment. Toutefois, il convient de noter que ces dimensions ne sont pas limitatives puisque l'on peut créer des barreaux de quelques micromètres de longueur et de diamètre, et à l'inverse créer des barreaux de quelques mètres et de plusieurs centimètres de diamètre.

Ce barreau hétérogène en composition peut être réalisé de plusieurs manières.

Il peut être réalisé à l'aide d'un moule parallélépipédique ou cylindrique, dans lequel sont introduits au moins deux constituants purs (tel que par exemple dans le domaine des métaux: le fer , le cobalt, l' aluminium, le cuivre, et tel que par exemple dans le domaine des oxydes métalliques: $Al_2O_3$, $Ag_2O$, $MnO_2$, $ZnO_2$, $Y_2O_3$, $Yb_2O_3$, $Gd_2O_3$, et tel que par exemple dans le domaine des matériaux minéraux thermiquement stables: NaCl, $CaF_2$, $YF_3$) ou au moins deux mélanges de compositions différentes, ces mélanges (tel que par exemple $Y_2O_3$-$Gd_2O_3$, $Y_2O_3$-$Yb_2O_3$-$Er_2O_3$, $Y_2O_3$-$YF_3$) étant ou non formés des mêmes constituants purs et se différenciant entre eux qualitativement et/ou quantitativement. Ces constituants purs et/ou mélanges de compositions différentes, appartenant au domaine d'existence d'un même matériau (c'est-à-dire que l'on est en présence d'un matériau monophasé), sont introduits dans le moule sous forme de couches successives progressivement compactées, formant ainsi des couches ou strates superposées de hauteurs identiques ou différentes, qui à leurs tours sont compressées sous presse.

A titre de rappel, un matériau est monophasé lorsqu'il est constitué d'une seule phase (la phase étant une partie homogène d'un système matériel). Par opposition au matériau monophasé, un matériau est dit polyphasé s'il est constitué de plusieurs phases. Par définition, une phase est homogène ou uniforme dans toute son étendue (voir I. Prigogine et R.Defay, Thermodynamique Chimique, Ed. Desoer, Liège, 1944, Tome I, page 7). Lors d'un changement de phase, le système, au sens de la thermodynamique, se présente comme la réunion de deux sous-systèmes homogènes possédant des propriétés distinctes. On appelle phase chacun des deux sous-systèmes. Plus précisément, une phase est une partie homogène, physiquement distincte, séparée des autres parties du système par une surface définie. La phase sous laquelle un système se présente est déterminée par la connaissance d'un certain ensemble de paramètres intensifs: température, pression, champ électrique ou magnétique, composition, structure ( voir comme référence Encyclopaedia Universalis, France, vol. 12, 1980, page 923).

Chaque constituant formant le matériau hétérogène (à savoir le barreau hétérogène) peut se présenter sous la forme d'une poudre ou sous la forme d'une matière fondue broyée après solidification. Chaque mélange est préalablement rendu homogène (par un mixage des poudres ou par une fusion qui est, dans ce cas, suivie d'une solidification puis d'un broyage) avant son introduction dans le moule.

Après le démoulage et un traitement thermique de frittage à température et pression adéquates, le barreau hétérogène et fritté est découpé selon un parallélépipède ou un cylindre.

Cette découpe peut être perpendiculaire à l'interface séparant les couches successives. Elle peut être également pratiquée de telle sorte que les plans de séparation des couches successives et de compositions différentes, initialement horizontaux dans le barreau hétérogène fritté, deviennent après découpe, des plans de séparation entre lesdites couches en position oblique dans le parallélépipède ou cylindre découpé.

Ainsi, le barreau hétérogène fritté résultant de la découpe (à savoir le barreau hétérogène fritté découpé) est constitué de strates ou couches de compositions différentes disposées soit horizontalement, soit en position oblique.

Le barreau hétérogène peut également être réalisé par association de barreaux de différentes compositions, chacun étant homogène en composition (c'est-à-dire formé chacun d'un unique constituant) et étant préalablement formé par frittage ou par fusion. Ces barreaux homogènes en composition et retaillés aux dimensions et formes désirées pourront être associés par simple contact de leurs faces ou imbriqués les uns dans les autres. Ils pourront être, le cas échéant, maintenus associés ou imbriqués, par le creuset servant d'enceinte lors de la fusion de zone selon la technique de la fusion de zone ou par un frittage effectué à température et pression adéquates, pour former un barreau hétérogène assemblé.

Par exemple, ces barreaux homogènes en composition peuvent être écornés parallèlement (association en biais ou en coin desdits barreaux par simple contact des plans de coupe de leur face écornée), c'est-à-dire par découpe d'un angle sur l'un des barreaux et découpe de l'angle complémentaire sur le barreau adjacent ou par frottement de deux barreaux ou plaques (à savoir par l'effritement de l'un par rapport à l'autre), ou ils peuvent être façonnés de façon à être imbriqués en association mâle-femelle, ou en tout autre forme voulue, selon des méthodes connues. Ils pourront être maintenus associés ou imbriqués, par une soudure effectuée soit par un frittage in situ à température et pression adéquates, soit par un collage à l'aide d'une matière appropriée, pour former un barreau hétérogène assemblé.

Ainsi, les barreaux hétérogènes en composition peuvent être réalisés selon des méthodes connues, telles que celles décrites précédemment, ou peuvent résulter selon l'invention d'une découpe particulière selon un parallélépipède ou un cylindre d'un matériau hétérogène, stratifié et fritté.

Sur le barreau hétérogène fritté tel qu'il est obtenu après découpe (c'est-à-dire le barreau hétérogène fritté découpé), constitué d'au moins deux constituants qui peuvent être des métaux ou des composés chimiques solides, ou sur le barreau hétérogène assemblé tel que défini précédemment, est réalisée une fusion de zone selon la technique de la fusion de zone (fondue) ou de zone flottante [voir, par exemple, W.G. Pfann, Fusion de Zone, J.Wiley N-Y 1958 ou L'art et la Science du développement des cristaux, J.Wiley et fils, Inc., N-Y, 3ème édition, 1996, pages 347-361, 389, 392 ou encore R.S.Feigelson, W.L. Kway, R.K. Route, Proc. SPIE Arlington-virginia, 484, 1984, 133].

La fusion de zone, réalisée selon les deux techniques citées précédemment, provoque une évolution progressive de la composition du liquide constituant la zone fondue, en raison de la solubilisation (dissolution ou fusion) progressive et en proportions variables des constituants formant le barreau hétérogène fritté tel qu'obtenu après découpe ou hétérogène assemblé. L'évolution de la composition du liquide constituant la zone fondue induit une évolution de la composition du solide cristallisé ou vitreux qui se forme lors du déplacement de la partie liquide le long du barreau hétérogène fritté tel qu'obtenu après découpe ou hétérogène assemblé.

A partir du barreau hétérogène formé de constituants rendus miscibles par fusion de zone, une fibre monophasée monocristalline ou polycristalline ou vitreuse est élaborée lors du tirage de ladite fibre à partir de la zone fondue.

Les techniques de fusion de zone ou de zone flottante appliquées à des barreaux hétérogènes en composition sont connues de l'art antérieur [voir par exemple, R. Cohen-Adad, J.-J. Barthélémy et J. Mack, C.R. Acad. Sci. Paris, 280 (1975), 1477]. L'article mentionné ci-dessus enseigne que lorsque la fusion de zone est réalisée sur un barreau hétérogène, formé de deux constituants A et B, la composition g du liquide varie de façon continue depuis g=0 (A pur) jusqu'à une limite qui dépend des quantités relatives de A et B dans le barreau, de la longueur du barreau et de la longueur de la zone fondue. La méthode a été appliquée dans le but d'obtenir le spectre d'un diagramme de phases par un examen micrographique qui permet de suivre l'évolution de morphologies des différentes phases observées et d'en déduire la nature des transitions de phases.

Cette technique de la fusion de zone est particulièrement bien adaptée à l'élaboration de fibres selon l'invention au même titre que la technique de la fusion par zone flottante qui est une variante de celle-ci.

Selon la technique de la fusion de zone ou de la zone flottante, une étroite zone du barreau hétérogène fritté tel qu'il a été obtenu après découpe ou hétérogène assemblé est fondue par chauffage (chauffage inductif ou résistif, rayonnement laser focalisé etc...) et est déplacée le long dudit barreau par un mouvement de translation relatif du moyen de chauffage et dudit barreau.

Selon la technique de la fusion par zone flottante, une zone fondue est créée entre deux barreaux verticaux, l'un barreau-source et l'autre barreau pouvant le cas échéant servir de germe pour obtenir la cristallisation de la fibre à gradient de composition.

Selon ces deux techniques, l'enceinte de travail dans laquelle sera élaborée la fibre monocristalline ou polycristalline ou vitreuse à gradient de composition permet une élaboration sous atmosphère contrôlée (oxydante, inerte ou réductrice) et permet de maintenir une pression inférieure ou supérieure à la pression atmosphérique.

Il convient toutefois de noter que la technique de la fusion par zone flottante est bien adaptée aux céramiques, en particulier aux céramiques hautement réfractaires, parce qu'elle évite l'utilisation d'un creuset à l'origine de forte réactivité et de contamination possible. Cette technique permet en outre d'utiliser des matières premières de très grande pureté sans les contaminer.

Les appareillages conçus pour l'utilisation de la technique de la fusion de zone ou de zone flottante font partie de l'art antérieur.

Ainsi, lors de la préparation d'une fibre monophasée monocristalline ou polycristalline ou vitreuse à gradient de composition ($F_G$) selon l'étape a) du procédé de l'invention, on effectue une fusion locale du matériau selon l'une ou l'autre des techniques citées précédemment, suivie d'un tirage de ladite fibre de façon à induire un gradient de composition. Au cours du déplacement de la zone fondue, la fusion, tranche par tranche, du barreau hétérogène fritté tel qu'il a été obtenu après découpe ou du barreau hétérogène assemblé entraîne une évolution progressive de la composition du liquide constituant la zone fondue par solubilisation (dissolution ou fusion) progressive et en quantités variables des constituants composant ledit barreau et induit un changement progressif de la composition de la fibre monocristalline ou polycristalline ou vitreuse formée par le déplacement de la zone fondue.

La fibre monophasée monocristalline ou polycristalline ou vitreuse à gradient de composition peut être élaborée jusqu'à l'obtention de la longueur souhaitée (de quelques millimètres à plusieurs dizaine de centimètres, voire plus d'un mètre). Les dimensions de cette fibre dépendent de la technique de la fusion de zone employée pour l'élaborer et peuvent parfois dépendre des dimensions des appareillages de mesure utilisés.

Selon les étapes b) et c) du procédé de l'invention, l'évolution de la composition tout au long de la fibre ($F_G$) ainsi que l'évolution d'au moins une caractéristique physique souhaitée tout au long de la même fibre, peuvent être déterminées par des techniques d'analyse connues en soit.

Dans le cas d'une fibre monophasée monocristalline ou polycristalline ou vitreuse à gradient de composition dont les dimensions sont adaptées aux appareillages de mesure, la détermination de l'évolution de la composition et d'au moins une caractéristique souhaitée se fera point par point tout au long de ladite fibre.

La mesure de la composition d'une fibre à gradient de composition est réalisée à l'aide de techniques d'analyse quantitative appropriées. Ces techniques permettent de suivre l'évolution de la concentration des différents constituants tout au long de la fibre à partir d'une origine notée 0 définie de façon arbitraire sur ladite fibre et ceci par une mesure point par point non destructive le long de la fibre en question. Pour pouvoir suivre l'évolution de la composition tout au long de la fibre, on définit donc sur la fibre une origine notée 0 (point O défini à priori sur la fibre) choisie de façon arbitraire sur ladite fibre. Ainsi, on peut établir, sous la forme d'une courbe, l'évolution de la composition point par point, de façon quasiment continue, tout au long de la fibre en fonction de la distance parcourue par rapport à l'origine choisie arbitrairement sur la fibre (point 0).

La fibre n'étant pas altérée par les mesures effectuées pour déterminer sa composition à l'aide de la technique d'analyse choisie, elle peut donc à nouveau faire l'objet de nouvelles analyses afin de déterminer insitu au moins une des caractéristiques physiques souhaitées et mesurables. Par conséquent, on peut mesurer point par point tout au long de la même fibre, selon une technique d'analyse appropriée, la réponse physique d'au moins cette caractéristique physique souhaitée à partir d'une nouvelle origine notée 0' définie à priori sur ladite fibre. Le positionnement d'une origine notée O (point 0) définie à priori sur une fibre monocristalline ou polycristalline ou vitreuse étant très difficile à retrouver avec exactitude, une nouvelle origine notée O' (point 0') définie de façon arbitraire est choisie le long de la même fibre afin de suivre l'évolution d'au moins une caractéristique physique désirée le long de ladite fibre. Par conséquent, les mesures de la réponse physique d'au moins une caractéristique physique souhaitée, effectuées point par point le long de la fibre vont permettre l'établissement d'une courbe réponse donnant l'évolution de la caractéristique physique désirée en fonction de la distance, à savoir la distance parcourue le long de la fibre par rapport à une origine arbitraire 0' choisie sur ladite fibre.

Ainsi, selon le procédé de l'invention des mesures point par point tout au long du matériau soumis à analyse peuvent être effectuées, et ceci de manière quasiment continue. Par le choix de méthodes d'analyse appropriées, il est envisageable de suivre l'évolution de la composition de la fibre ainsi que l'évolution d'au moins une caractéristique physique souhaitée de façon quasiment continue par des mesures possibles par exemple tous les 20 micromètres le long de la fibre à partir de l'origine choisie de façon arbitraire sur ladite fibre, voire tous les 3 à 4 micromètres. En utilisant par exemple comme technique d'analyse quantitative une microsonde pour déterminer l'évolution de la composition d'une fibre selon l'invention, on peut envisager des mesures possibles tous les 3 à 4 micromètres sur une fibre longue de 8 millimètres au plus.

Ainsi, plusieurs centaines de mesures peuvent être effectuées sur la longueur de fibre préparée selon l'invention permettant l'analyse de façon quasiment continue des phénomènes physiques et chimiques liés au gradient de composition de la fibre et au traitement thermique subi.

Après la mesure de la composition (non destructive), effectuée point par point, puis d'au moins une caractéristique physique souhaitée pour ledit matériau, c'est-à-dire la fibre monocristalline ou polycristalline ou vitreuse préparée selon l'étape a) du procédé de l'invention, on peut déterminer la composition idéale ($C_I$) correspondante à la valeur optimale de la caractéristique physique souhaitée ($R_I$) selon les étapes d) , e) et f) du procédé de l'invention. Comme il l'a été souligné précédemment le positionnement de l'origine définie à priori sur une fibre monocristalline ou polycristalline ou vitreuse est très difficile à retrouver avec exactitude. C'est pourquoi deux origines, le point 0 et le point 0' correspondant respectivement à la mesure de la composition ($C_0$) pour l'origine 0 le long de ladite fibre et à la mesure de la réponse physique ($R_{0'}$) correspondant à l'origine 0' le long de la même fibre obtenue pour la caractéristique physique souhaitée, ont été définies arbitrairement sur la fibre à analyser.

Puis est établi la corrélation entre la composition ($C_0$) ayant l'origine arbitraire (0) sur la courbe de composition et la réponse physique ($R_{0'}$) ayant l'origine arbitraire (0') sur la courbe de réponse physique obtenue pour la caractéristique physique souhaitée sur la même fibre.

Pour établir cette corrélation, on prépare une fibre de référence ($F_R$) de composition homogène ($C_{FR}$) sur laquelle on mesure exactement en au moins un point la composition ($C_{FR}$) et la réponse physique ($R_{FR}$) correspondante obtenue pour la caractéristique physique souhaitée sachant que le choix de la composition homogène pour la fibre de référence ($F_R$) de l'étape d) du procédé se fait dans une zone de composition de la fibre

($F_G$), pour laquelle la réponse physique est univoque au sens mathématique du terme. Ainsi, selon l'étape e) du procédé de l'invention cette fibre de référence à composition homogène, à savoir constante tout au long de la fibre, par une mesure exacte en au moins un point de la composition ($C_{FR}$) et de la réponse physique ($R_{FR}$) correspondante, va permettre pour la fibre ($F_G$) la mise en correspondance de la courbe de composition ($C_{FG}$) ayant pour origine arbitraire (0) et de la courbe de réponse physique ($R_{FG}$) ayant pour origine arbitraire (0'), et ceci par la mise en concordance, sur une même verticale des valeurs ($C_{FR}$) (reportée sur la courbe de composition de $F_G$) et ($R_{FR}$) (reportée sur la courbe de réponse physique de $F_G$). Les courbes de la composition ($C_{FG}$) de la fibre ($F_G$) et de la réponse physique ($R_{FG}$) obtenue pour la caractéristique physique souhaitée sur la même fibre ayant été mises en correspondance, on peut alors déterminer par une lecture directe sur la courbe de composition quelle est la composition idéale ($C_I$) de la fibre analysée ($F_G$) correspondante à la valeur optimale ($R_I$) de la réponse physique de la caractéristique physique souhaitée. Par conséquent, à partir de la réponse physique la plus favorable ($R_I$) de ladite caractéristique, on va pouvoir définir sur la courbe de composition, la composition de la fibre ($F_G$) qui s'avérera optimale, en d'autre terme idéale, pour au moins une caractéristique physique souhaitée elle-même optimisée.

Comme techniques d'analyse quantitative permettant de déterminer la composition point par point tout au long de la fibre sans destruction de ladite fibre, on peut citer par exemple, la microsonde. A titre de techniques d'analyse permettant de déterminer l'évolution d'au moins une caractéristique physique souhaitée pour un matériau, on peut citer par exemple, la microscopie optique ou la microscopie électronique à balayage. A titre d'exemple, on peut envisager la détermination de l'évolution de la microstructure par microscopie optique sur platine chauffante à la condition qu'aucun des constituants de la fibre ne soit volatil et que la température soit toujours inférieure à la température de fusion de la fibre, sachant que cette température varie avec la composition de ladite fibre. Il s'agit par conséquent de méthodes de mesure ponctuelles non destructives de la fibre.

A titre de caractéristique (s) physique(s) non destructive(s) mesurable(s) point par point tout au long de la fibre, on peut citer par exemple des propriétés physiques telles que les propriétés optiques, magnétiques, électriques et mécaniques. Comme propriétés électriques, on peut citer par exemple la conductivité électrique, la résistivité ou encore la constante diélectrique. A titre de propriétés mécaniques, on peut envisager par exemple la microdureté, notamment d'un alliage métallique. A titre de propriétés optiques, on peut citer par exemple les spectres d'absorption, d'émission ou de luminescence, l'indice de réfraction, l'indice optique ou encore la durée de vie d'un ion excité. On peut également mentionner la conductibilité thermique, la microstructure ou la nanostructure comme caractéristique physique souhaitée mesurable selon l'invention. En effet, la détermination de la microstructure d'une fibre par des mesures ponctuelles le long de cette fibre ainsi que la détermination de l'évolution de sa composition selon le procédé de l'invention peuvent permettre de trouver les conditions de dévitrification des céramiques dans le domaine des porcelaines par exemple ou l'amélioration des propriétés mécaniques d'un alliage notamment par optimisation de la taille des grains (affinage) des métaux précieux dans le domaine des alliages utilisés dans la bijouterie ou dans l'aérospatial par exemple.

Il convient de noter que cette énumération des propriétés physiques mesurables selon l'invention ne saurait en aucun cas être interprétée de manière limitative.

Dans le cas d'une fibre monophasée monocristalline ou polycristalline ou vitreuse à gradient de composition dont les dimensions sont supérieures aux limites imposées par les appareillages de mesure, ladite fibre pourra être tronçonnée en fibre de dimension adéquate et chaque partie tronçonnée sera analysée comme décrit précédemment.

Par ailleurs, comme il ressort des exemples présentés plus loin, le procédé selon l'invention présente l'avantage de permettre et d'obtenir une optimisation rapide de matériaux complexes par la détermination de la composition idéale correspondant à une ou plusieurs caractéristiques physiques souhaitées optimisées. Ces exemples sont donnés afin d'illustrer des modes d'exécution particuliers de l'invention et ne sauraient être interprétés de manière restrictive.

Les avantages propres à l'emploi du procédé de l'invention sont les plus évidents lorsque le matériau optimisé de composition idéale résultant des étapes de préparation et d'analyses du procédé et comportant la ou les caractéristiques physiques optimales souhaitées, est obtenu. En effet, le procédé selon l'invention permet de réduire le temps d'expérimentation nécessaire à la mise au point d'un matériau de l'invention, tout en étant d'un emploi économique. De ce fait, le procédé ainsi que les matériaux selon l'invention peuvent trouver un domaine d'application fort étendu. De plus, de tels matériaux peuvent présenter un vif intérêt économique dans de nombreux secteurs d'activité industrielle notamment au vu d'une réduction substantielle du coût engendrée d'une part par l'élaboration rapide des dits matériaux et d'autre part par l'exploitation de ceux-ci.

Le procédé selon l'invention peut s'appliquer à l'élaboration de fibres monophasées polycristallines à gradient de composition et, à ce titre, peut concerner de nombreux domaines industriels. A titre illustratif, le procédé selon l'invention concerne l'industrie métallurgique et plus particulièrement le domaine de la recherche d'alliages. En effet, les caractéristiques mécaniques d'un alliage sont essentiellement déterminées par l'état de la matière métallique et en particulier par la structure et la taille de ses grains. Or, les différentes opérations de forgeage, de laminage, de tréfilage, d'étirage réalisées pour induire une forme ou des dimensions de grains au sein d'un alliage de coulée conduisent souvent à une déformation desdits grains. Actuellement un traitement thermique dit de recuit (maintien de l'alliage à une température comprise entre 400 et 800°C pendant quelques minutes voire plusieurs heures dans certains cas) est utilisé pour palier à ces inconvénients. Cependant durant ce traitement, les caractéristiques physiques de l'alliage comme par exemple la résistance à la traction, la dureté ou la ductilité peuvent être modifiées. Toute transformation mécanique pourra se traduire par exemple pour un alliage de bijouterie par la formation d'un effet «peau d'orange» dû au grossissement excessif des grains, voire même par des fissurations dans certains alliages à la suite d'une décohésion des grains. La mise au point du traitement de recuit ainsi que l'ajout d'affineurs de grains notamment dans le domaine de la bijouterie permettent de réduire, de manière plus ou moins importante, la taille desdits grains et de maintenir cette dernière à des valeurs limitées maîtrisées lors des traitements thermiques. Mais d'une façon générale, il reste difficile de corréler la composition à la microstructure et à l'évolution de cette microstructure lors de traitements thermiques, notamment dans le domaine de la bijouterie où la faible taille des grains est essentielle pour la fabrication de fils fins par exemple. Du fait de la complexité de la mise au point d'un alliage en termes de relations composition-microstructure-caractéristique physique, le procédé de l'invention est parfaitement bien adapté à cette mise au point, car l'influence conjuguée de la composition et du traitement thermique peut être déterminée sur une même fibre à gradient de composition élaborée selon l'invention et ainsi permettre l'optimisation rapide de tels alliages.

Par ailleurs, l'invention concerne aussi l'application des matériaux optimisés de composition idéale résultant du procédé de l'invention et comportant la caractéristique physique souhaitée, rendue optimale.

Les matériaux optimisés résultant du procédé de l'invention peuvent également trouver un emploi avantageux aussi bien dans l'industrie métallurgique et le domaine de la bijouterie que dans l'industrie de la microélectronique, dans l'aérospatial ou encore dans l'industrie automobile ou autre. L'industrie microélectronique, par exemple, utilise des céramiques techniques en raison de leurs exceptionnelles propriétés électromagnétiques, de leur conductivité électrique ou de leur pouvoir isolant, de leur résistance en température ou de leur inertie chimique. L'aérospatial tout comme l'industrie de l'armement est en permanence à la recherche de matériaux résistants à des chocs thermiques toujours plus hauts en température. L'invention peut s'appliquer au domaine de transmission optique à travers les fibres optiques vitreuses.

L'invention est illustrée de façon plus détaillée à l'aide des exemples présentés ci-après, dans lesquels les abréviations ont le sens usuel dans l'art.

## Description des dessins

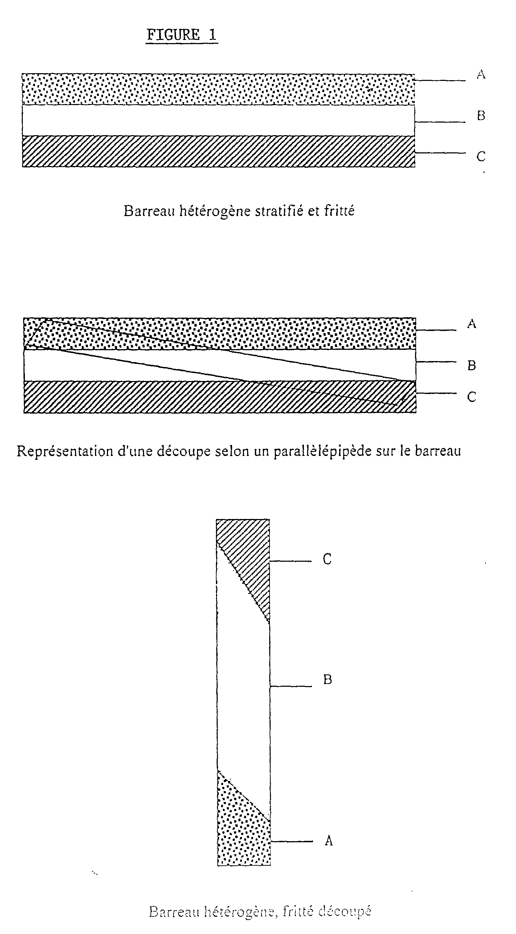

- La Figure 1 représente une vue faciale d'un barreau hétérogène, stratifié et fritté, constitué de trois constituants A, B et C, ainsi qu'une vue faciale de ce barreau où est représentée une découpe selon un parallélépipède et une vue faciale du barreau découpé obtenu.
- La Figure 2 représente le schéma du fractionnement du matériau à base d'oxyde de gadolinium (Gd$_2$O$_3$) en domaines de composition, ce schéma ayant trait à l'expérience de l'exemple 1.
- La Figure 3 représente les graphiques ayant trait à l'expérience de l'exemple 1, ces graphiques représentant, sous la forme de courbes, l'évolution de la composition en ytterbium pour les fibres A, B et C.
- La Figure 4 représente les graphiques ayant trait à l'expérience de l'exemple 1, ces graphiques représentant sous la forme de courbes, l'évolution de la teneur en ions dopants et l'évolution de la durée de vie de fluorescence.
- La Figure 5 représente le graphique ayant trait à l'expérience de l'exemple 1, ce graphique représentant sous la forme d'une courbe, la corrélation existant entre la durée de vie de fluorescence du niveau excité de l'ion Yb$^{3+}$ et la teneur en ions dopants Yb$^{3+}$.
- La Figure 6 représente les graphiques ayant trait à l'expérience de l'exemple 2, ces graphiques représentant, sous la forme de courbes, l'évolution en composition des deux fibres 1 et 2,et une projection sur le plan des compositions de deux coupes isopléthiques d'un diagramme isoternaire.
- La Figure 7 représente le graphique ayant trait à l'expérience de l'exemple 2, ces graphiques représentant, sous la forme de courbes, l'évolution en composition de fibre 2 et l'évolution de la durée de vie de l'ion excité Er$^{3+}$ sur la fibre 2.
- La Figure 8 représente les graphiques ayant trait à l'expérience de l'exemple 2, ces graphiques représentant, sous la forme de courbes, l'évolution en composition de la fibre 1 et l'évolution de la durée de vie de l'ion excité Er$^{3+}$ sur la fibre 1.
- La Figure 9 représente le graphique ayant trait à l'expérience de l'exemple 2, ce graphique représentant, sous la forme de courbes, la corrélation existant entre la durée de vie de l'ion Er$^{3+}$ et les compositions des deux fibres 1 et 2.
- La Figure 10 représente le graphique ayant trait à l'expérience de l'exemple 3, ce graphique représentant, sous la forme de courbes, l'évolution de la composition et l'évolution de la microdureté vickers de la fibre pour une solution solide de cobalt dans le fer.
- La Figure 11 représente le graphique ayant trait à l'expérience de l'exemple 3, ce graphique représentant, sous la forme de courbes, l'évolution de la microdureté vickers en fonction de la composition en cobalt de la fibre.

## Exemple 1

Une optimisation des propriétés optiques, notamment la durée de vie du niveau excité d'un ion dopant dans un matériau peut être réalisée en fonction de la teneur en ions dopants.

Lors d'une application laser du matériau, l'effet laser ou l'émission laser, dont la longueur d'onde dépend de la nature de l'ion dopant, est optimisée (à savoir la puissance de l'émission laser sera optimisée) à travers l'optimisation de la section efficace de l'émission stimulée de l'ion dopant.

Cette section efficace de l'émission stimulée de l'ion dopant est déterminée à partir d'une relation liant l'indice optique, la largeur à mi-hauteur de la raie d'émission considérée et la probabilité radiative spontanée qui est directement liée à la durée de vie de l'ion dopant.

Cette relation liant la section efficace de l'émission stimulée aux paramètres indice optique du milieu (n), largeur de la bande d'émission à mi-hauteur (Δλ), durée de vie d'un ion excité (τ) est la suivante :

$$\sigma = \frac{\lambda^2}{8\Pi c n^2}\,\frac{1}{\Delta\lambda}\,P$$

Dans cette relation, c est la vitesse de la lumière et P représente la probabilité d'émission radiative spontanée. Cette dernière valeur est calculable à partir de la théorie de Judd-Ofelt et en première approximation, on peut considérer qu'elle varie comme l'inverse de la durée de vie (1/τ) (voir par exemple, Fuxi Gan, matériaux laser, publication scientifique du monde Co. ISBN 981 02 1580 0, (1995), p. 77).

Ces trois paramètres, mesurables in situ, permettent d'optimiser l'effet laser.

L'exemple présenté ici concerne l'optimisation d'un de ces trois paramètres, à savoir la durée de vie du niveau excité d'un ion dopant Yb$^{3+}$ en fonction de sa teneur dans une matrice constituée par le système monocristallin oxyde de gadolinium et ion dopant Yb$^{3+}$, constituant un matériau trouvant des applications dans la construction de laser tout solide.

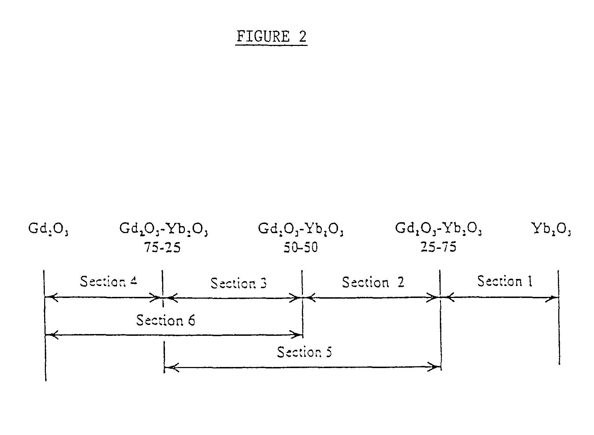

Pour obtenir une meilleure précision dans l'optimisation de la teneur en ions Yb$^{3+}$ dans le matériau Gd$_2$O$_3$, l'axe des compositions a été fractionné en domaines avec des recouvrements entre les domaines de composition comme le montre la figure 2.

Selon la technique de la fusion de zone flottante, six fibres monocristallines sont élaborées selon l'étape a) du procédé de l'invention, à partir de deux barreaux dont les compositions respectives en oxyde de gadolinium (Gd$_2$O$_3$) sont les suivantes :

- Fibre A : barreau-source 100 at% de $Gd_2O_3$ et barreau-germe 50 at% de $Gd_2O_3$ (soit $Gd_2O_3$)-$Yb_2O_3$=50-50 at%), représentant la section 6 du domaine fractionné.
- Fibre B : barreau-source 75 at% de $Gd_2O_3$ (soit $Gd_2O_3$-$Yb_2O_3$=75-25 at%) et barreau-germe 25 at% de $Gd_2O_3$ (soit $Gd_2O_3$-$Yb_2O_3$=25-75 at%), représentant la section 5 du domaine fractionné.
- Fibre C : barreau-source 100 at% de $Gd_2O_3$ et barreau-germe 75 at% de $Gd_2O_3$ (soit $Gd_2O_3$-$Yb_2O_3$=75-25 at%), représentant la section 4 du domaine fractionné.
- Fibre D : barreau-source 50 at% de $Gd_2O_3$ (soit $Gd_2O_3$-$Yb_2O_3$=50-50 at%), et barreau-germe 25 at% de $Gd_2O_3$ (soit $Gd_2O_3$-$Yb_2O_3$=25-75 at%), représentant la section 2 du domaine fractionné.
- Fibre E : barreau-source 25 at% de $Gd_2O_3$ (soit $Gd_2O_3$-$Yb_2O_3$=25-75 at%), et barreau-germe 0 at% de $Gd_2O_3$ (soit $Gd_2O_3$-$Yb_2O_3$=0-100 at%), représentant la section 1 du domaine fractionné.
- Fibre F : barreau-source 75 at% de $Gd_2O_3$ (soit $Gd_2O_3$-$Yb_2O_3$=75-25 at%), et barreau-germe 25 at% de $Gd_2O_3$ (soit $Gd_2O_3$-$Yb_2O_3$=25-75 at%), représentant la section 5 du domaine fractionné.

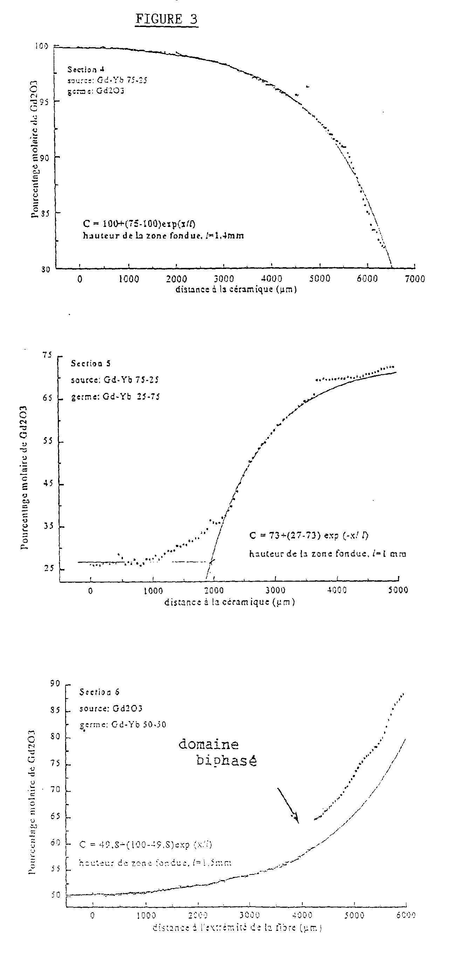

Pour chaque fibre, l'évolution de la composition en ytterbium a été mesurée par microsonde. La figure 3 donnée à titre d'exemple est relative aux fibres A, B et C, elle représente les courbes obtenues lors de l'évolution de la composition en fonction de la distance mesurée en micromètres par rapport à une origine arbitrairement choisie sur la fibre.

Sur la fibre C (section 4), la teneur en ion $Yb^{3+}$ évolue de façon continue. Cette évolution continue confirme l'existence d'une solution solide riche en gadolinium.

Sur les courbes relatives aux fibres A (section 6) et B (section 5), un décrochement est observé approximativement entre 30 et 42 at% en $Yb_2O_3$ correspondant à un domaine biphasé. Au-delà de 42 at% en Yb, on retrouve à nouveau un domaine de solution solide. La mesure des propriétés physiques sera effectuée sur la partie monophasée de la fibre.

Après la détermination de la composition en ytterbium des fibres (voir figure 3 pour les fibres A, B et C), une mesure in situ de la durée de vie de fluorescence du niveau excité de l'ion $Yb^{3+}$ a été effectuée.

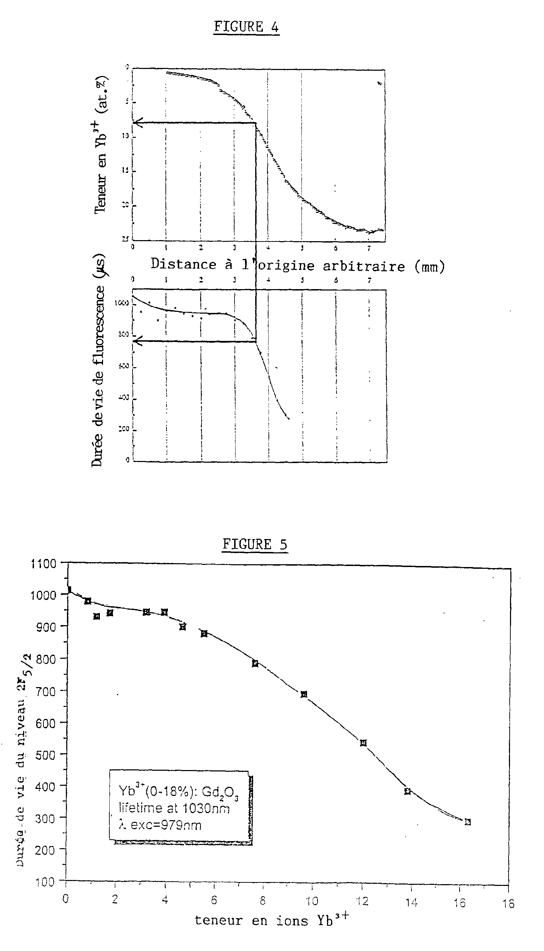

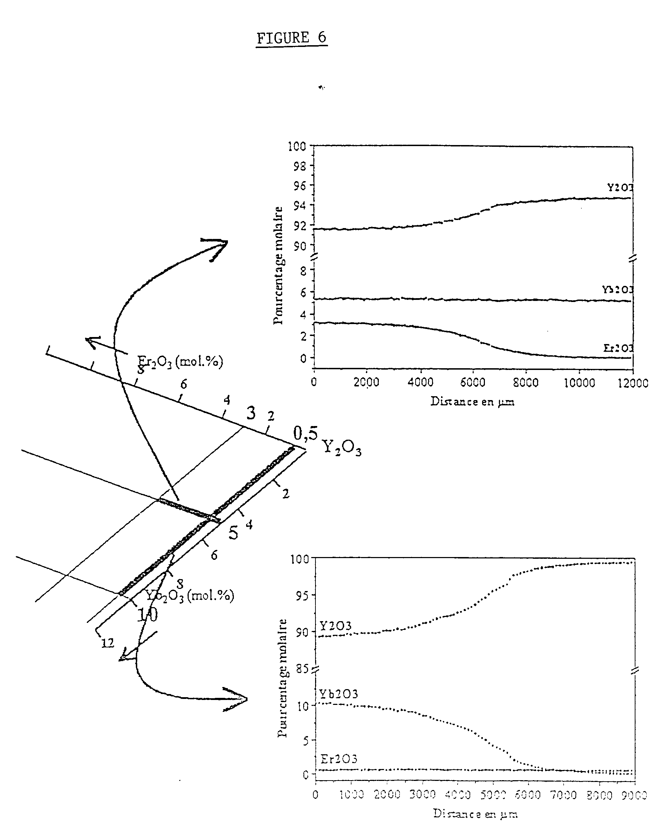

Selon le procédé de l'invention, on détermine la corrélation composition-réponse physique de la caractéristique physique. On peut ainsi déterminer la durée de vie du niveau excité en fonction de la teneur en ions dopants. Les figures 4 et 5 représentent la corrélation existant entre la durée de vie de fluorescence du niveau excité (en microsecondes) et la teneur en ions dopants $Yb^{3+}$ (en at%).

D'après les courbes représentées sur la figure 4, la durée de vie du niveau excité est inférieure à 800 μs (environ 80% de la durée de vie à grande dilution) à partir d'une teneur de 8 at% en ion $Yb^{3+}$. Au-delà de cette teneur, on constate une décroissance rapide de la durée de vie du niveau excité avec l'augmentation du taux de dopage. On peut en déduire que, pour une teneur inférieure à 8 at% de $Yb^{3+}$, la durée de vie de l'ion excité permet une application laser du matériau analysé. Au-dessus de 8 at% de $Yb^{3+}$, les applications laser seront à priori moins favorables. Par contre, la connaissance de l'évolution de la durée de vie de l'ion dopant pour une teneur supérieure à 8 at% permet d'accéder à la connaissance des processus de transfert d'énergie entre les ions $Yb^{3+}$ comme la luminescence coopérative entre les aggrégats d'ytterbium.

Exemple 2

Une optimisation des propriétés optiques, notamment de la durée de vie des ions dopants excités d'une fibre à base d'oxyde d'yttrium ($Y_2O_3$) codopée par des ions ytterbium et erbium ($Yb^{3+}$-$Er^{3+}$) a été réalisée à l'aide de deux fibres monocristallines, élaborées selon l'étape a) du procédé de l'invention c'est-à-dire selon la technique de la fusion par zone flottante et ayant les compositions suivantes :

- Fibre 1 à teneur fixe en Yb (5 at%) et à teneur variable en Er (comprise entre 0 et 3 at%).
- Fibre 2 à teneur fixe en Er (0,5 at%) et à teneur variable en Yb (comprise entre 0 et 10 at%).

Pour chaque fibre, l'évolution des teneurs en chacun des constituants ($Y_2O_3$, $Er_2O_3$, $Yb_2O_3$) les formant a été déterminée par microsonde et est représentée sous la forme d'une courbe dans la figure 6 en fonction de la distance mesurée en micromètres par rapport à une origine arbitrairement choisie sur la fibre.

Les deux fibres peuvent également être décrites en terme de coupes isopléthiques (c'est-à-dire coupe plane dans un diagramme de phase pour laquelle il existe une relation entre les compositions des constituants) dans un diagramme ternaire s'appuyant sur le pourcentage molaire des différents constituants présents, à savoir $Y_2O_3$, $Er_2O_3$ et $Yb_2O_3$ (figure 6).

Il convient de noter que dans les conditions d'élaboration des deux fibres, chaque fibre est obtenue monophasée et monocristalline, ce qui est tout à fait cohérent avec la similitude de structure cristalline des trois oxydes et des rayons ioniques des trois ions métalliques.

Après la détermination des compositions des deux fibres telles que représentées sur la figure 6, des mesures in situ de la durée de vie du niveau excité de l'ion $Er^{3+}$ ont été effectuées sur chacune des deux fibres afin de déterminer l'évolution de la caractéristique physique souhaitée à savoir la durée de vie de l'ion excité $Er^{3+}$.

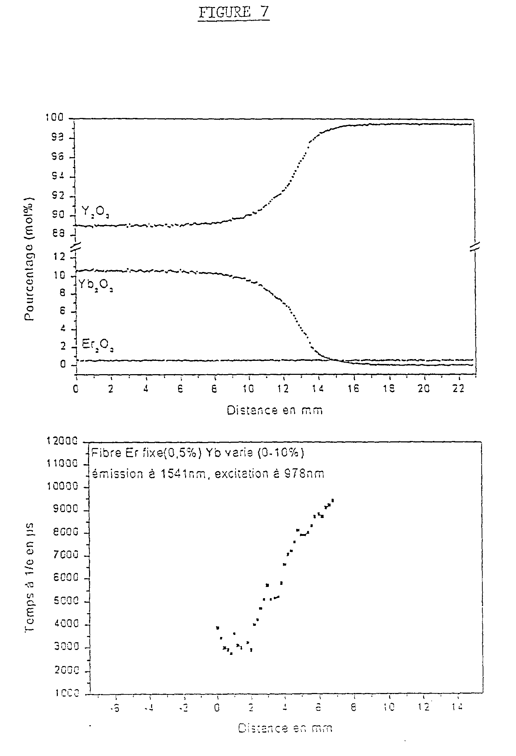

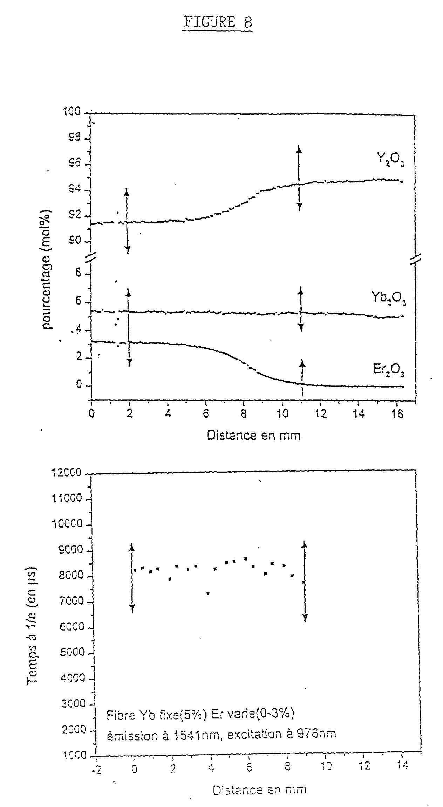

En effet, l'ion $Yb^{3+}$ a un rôle d'ion sensibilisateur à travers lequel l'énergie de vibration de la matrice $Y_2O_3$ est transmise à l'ion $Er^{3+}$ qui est l'ion émetteur. La durée de vie de l'ion émetteur $Er^{3+}$ a donc été mesurée in situ sur chaque fibre (figures 7 et 8) en fonction de la distance par rapport à une origine choisie de façon arbitraire le long de chaque fibre (origine arbitraire différente de celle choisie pour les mesures de compositions).

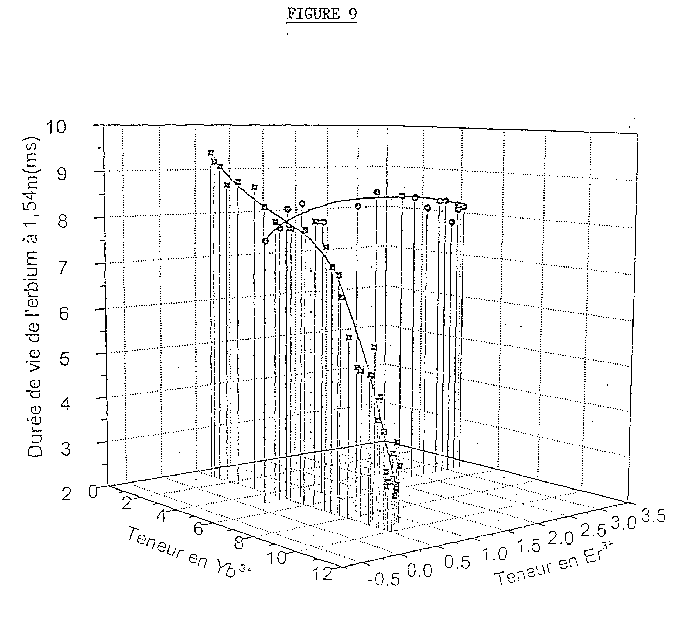

La corrélation durée de vie-composition a pu être établie et est représentée sur la figure 9. Ainsi, la surface utile pour une optimisation de la durée de vie de l'ion $Er^{3+}$ pour une application comme matériau laser et en fonction des compositions en ions $Er^{3+}$ et $Yb^{3+}$ peut être déterminée.

Ainsi, un nombre très limité d'échantillons, par exemple l'élaboration de quatre fibres monocristallines, peut conduire à une optimisation rapide des propriétés optiques en l'occurrence de la durée de vie d'un ion excité et définir le domaine de concentration optimale des ions sensibilisateurs et activateurs.

### Exemple 3

L'optimisation d'une caractéristique physique mesurable in situ telle que la microdureté vickers d'un alliage binaire fer-cobalt, a été réalisée à la suite de l'établissement d'une corrélation entre microstructure-composition-caractéristique physique mesurable.

Une fibre monophasée et polycristalline à gradient de composition ayant une teneur en cobalt comprise entre 73,5 et 0 at% a été préparée. En effet, dans le système binaire fer-cobalt, il existe une solubilisation à l'état solide de cobalt dans le fer qui s'étend entre 0 et 73,5 at% de cobalt et une solution solide de fer dans le cobalt qui s'étend de 85,2 à 100 at% de cobalt.

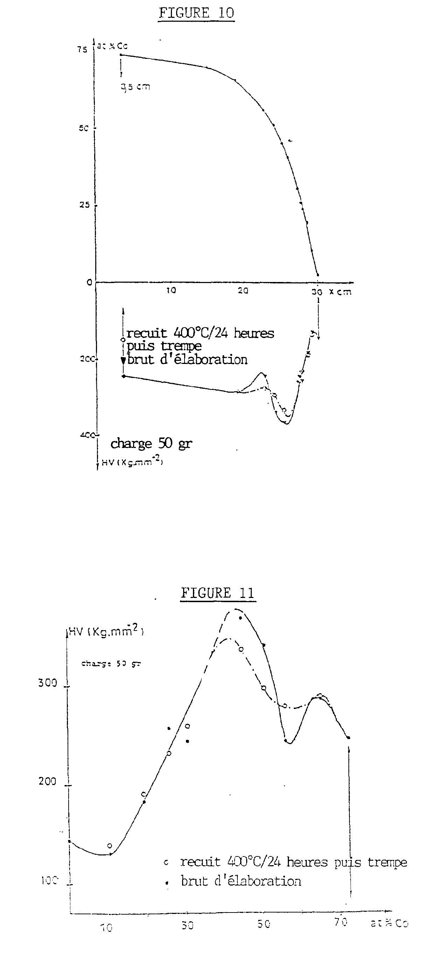

Des mesures de composition et de microdureté vickers ont été effectuées sur la même fibre monophasée élaborée entre 73,5 et 0 at% de cobalt. L'évolution de la composition et celle de la microdureté vickers en fonction de la distance parcourue par rapport à une origine choisie sur la fibre sont représentées sous la forme de courbes (figure 10). La distance parcourue a été normalisée de façon à ce que les extrémités des deux courbes soient cohérentes puisqu'elles expriment la microdureté vickers pour les deux compositions limites de la fibre : fer pur (c'est-à-dire 100 at% de fer) et 73,5 at% de cobalt (c'est-à-dire la limite supérieure de la solution solide). L'évolution de la microdureté vickers en fonction de la composition en cobalt de la fibre (figure 11) peut être déduite de l'exploitation des courbes de la figure 10.

La composition idéale ($C_I$) de la fibre peut être déterminée par lecture directe de la composition correspondant à la valeur optimale ($R_I$) de la microdureté vickers, sur la courbe représentée figure 11 en fonction de l'application recherchée, l'évolution de la dureté vickers en fonction de la composition en cobalt de la fibre (figure 11).

Pour chaque composition d'alliage fer cobalt, la valeur de la microdureté vickers est obtenue par lecture directe sur la figure 11.

Par exemple, pour une application nécessitant une microdureté importante, une composition correspondant à une teneur de 42 at% en cobalt de l'alliage Fe-Co conduira à la valeur optimale de la microdureté.

### Revendications

**1.** Procédé d'optimisation d'au moins une caractéristique physique souhaitée pour un matériau formé à partir d'au moins deux constituants, qui peuvent être des métaux ou des composés chimiques solides, rendus miscibles par fusion de zone, par détermination de la composition idéale ($C_I$) dudit matériau conduisant à la valeur optimale ($R_I$) de ladite caractéristique, **caractérisé en ce que** le procédé comporte les étapes suivantes :

(a) la préparation d'une fibre monophasée monocristalline ou polycristalline ou vitreuse à gradient de composition ($F_G$) continu, à partir d'un matériau formé d'au moins deux constituants, qui peuvent être des métaux ou des composés chimiques solides, élaborée selon une technique de fusion de zone;
(b) la détermination, sans destruction de la fibre, de la composition ($C_{FG}$) par une mesure point par point tout au long de ladite fibre et l'établissement de la courbe de composition correspondante ;
(c) la détermination sur la même fibre de la réponse physique ($R_{FG}$) d'au moins une caractéristique physique souhaitée par une mesure point par point, non destructive, et l'établissement de la courbe de la réponse physique correspondante ;
(d) la préparation d'une fibre de référence ($F_R$) de composition homogène($C_{FR}$), mettant en oeuvre les constituants, sur laquelle on mesure exactement en au moins un point la composition ($C_{FR}$) et la réponse physique ($R_{FR}$) correspondante à au moins une caractéristique physique souhaitée pour permettre l'établissement de la corrélation entre la composition ($C_{FG}$) et la réponse physique ($R_{FG}$) correspondante à au moins une caractéristique physique souhaitée de la fibre ($F_G$) ;
(e) la mise en correspondance pour la fibre ($F_G$) de la courbe de composition ($C_{FG}$) et de la courbe de la réponse physique ($R_{FG}$) d'au moins une caractéristique physique souhaitée par la mise en concordance, sur une même verticale des valeurs ($C_{FR}$) et ($R_{FR}$) reportées sur les courbes respectives de la fibre $F_G$ ;
(f) l'optimisation de la composition d'une fibre par lecture directe sur la courbe de composition de la fibre ($F_G$) de la composition idéale ($C_I$) correspondant à la valeur optimale ($R_I$) de la réponse physique de la caractéristique physique souhaitée.

**2.** Procédé selon la revendication 1, **caractérisé en ce que** la composition ($C_{FG}$) de la fibre ($F_G$) est déterminée par une technique d'analyse quantitative permettant d'établir, sous forme d'une courbe, l'évolution de la concentration des différents constituants, en fonction de la distance par rapport à une origine notée 0 arbitrairement choisie sur la fibre.

**3.** A titre de technique d'analyse quantitative selon l'une au moins des revendications 1 à 2, la microsonde.

**4.** Procédé selon l'une au moins des revendications 1 à 3, **caractérisé en ce que** la réponse physique ($R_{FG}$) d'au moins une caractéristique physique souhaitée est déterminée à l'aide de l'une des techniques d'analyse permettant l'établissement de la courbe réponse donnant l'évolution de la caractéristique physique en fonction de la distance par rapport à une origine notée 0' arbitrairement choisie sur la fibre.

**5.** Procédé selon l'une au moins des revendications 1 à 4, **caractérisé en ce que** le choix de la composition pour la fibre de référence ($F_R$) se fait dans une zone de composition de la fibre ($F_G$), pour laquelle la réponse physique est univoque.

**6.** A titre de caractéristique physique mesurable point par point selon l'une au moins des revendications 1 à 5, la microdureté, la microstructure, les spectres d'absorption, d'émission ou de luminescence, l'indice optique, l'indice de réfraction, la constante diélectrique, la conductibilité thermique.

**7.** Matériau selon l'une au moins des revendications 1 à 6, **caractérisé en ce que** le matériau est constitué par un mélange de métaux, d'oxydes métalliques ou autres matières minérales.

**8.** Matériau selon l'une au moins des revendications 1 à 7, **caractérisé en ce que** le matériau est un alliage métallique, une céramique industrielle ou un matériau minéral binaire ou d'ordre supérieur.

**9.** Matériau selon la revendication 8, **caractérisé en ce que** le matériau est une céramique hautement réfractaire.

**10.** Matériau selon l'une au moins des revendications 1 à 9, **caractérisé en ce que** le matériau est un barreau hétérogène assemblé ou un barreau hétérogène stratifié et fritté résultant d'une découpe particulière selon un parallélépipède ou un cylindre.

**11.** Matériau selon l'une au moins des revendications 1 à 10, **caractérisé en ce que** le barreau hétérogène fritté résultant de la découpe est un barreau hétérogène en composition, formé d'au moins deux constituants purs ou d'au moins deux mélanges de compositions différentes, ces mélanges étant formés ou non des mêmes constituants, appartennant au domaine d'existence du même matériau.

**12.** Matériau selon la revendication 11, **caractérisé en ce que** la découpe du barreau hétérogène et fritté est perpendiculaire à l'interface séparant les couches successives ou elle est pratiquée de telle sorte que les plans de séparation des couches successives et de compositions différentes, initialement horizontaux dans le barreau hétérogène fritté, deviennent après découpe, des plans de séparation entre lesdites couches en position oblique dans le parallélépipède ou cylindre découpé.

**13.** Matériau selon l'une au moins des revendications 1 à 12, **caractérisé en ce que** le barreau hétérogène et fritté résultant de la découpe est constitué de strates ou couches de compositions différentes disposées soit horizontalement, soit en position oblique.

**14.** Matériau résultant des étapes de préparation et d'analyses du procédé selon l'une au moins des revendications 1 à 13, **caractérisé en ce que** le matériau a une composition idéale correspondante à la caractéristique physique optimale souhaitée pour ledit matériau.

**15.** Utilisation d'un matériau selon la revendication 14, à titre de matériau optimisé.

**16.** Alliages métalliques ou céramiques industrielles, formé d'un matériau selon la revendication 14.

**17.** Céramiques industrielles selon les revendications 15 ou 16, sous la forme de céramiques hautement réfractaires, de céramiques utilisées dans la fabrication des porcelaines.

FIGURE 1

Barreau hétérogène stratifié et fritté

Représentation d'une découpe selon un parallélépipède sur le barreau

Barreau hétérogène, fritté découpé

FIGURE 2

$Gd_2O_3$       $Gd_2O_3$-$Yb_2O_3$       $Gd_2O_3$-$Yb_2O_3$       $Gd_2O_3$-$Yb_2O_3$       $Yb_2O_3$

75-25       50-50       25-75

Section 4    Section 3    Section 2    Section 1

Section 6

Section 5

FIGURE 3

Section 4
source: Gd-Yb 75-25
germe: Gd2O3

$C = 100 + (75-100)\exp(x/l)$
hauteur de la zone fondue, $l = 1,4$ mm

Pourcentage molaire de Gd2O3

distance à la céramique (μm)

Section 5
source: Gd-Yb 75-25
germe: Gd-Yb 25-75

$C = 73 + (27-73)\exp(-x/l)$
hauteur de la zone fondue, $l = 1$ mm

Pourcentage molaire de Gd2O3

distance à la céramique (μm)

Section 6
source: Gd2O3
germe: Gd-Yb 50-50

domaine
biphasé

$C = 49,8 + (100-49,8)\exp(x/l)$
hauteur de zone fondue, $l = 1,5$ mm

Pourcentage molaire de Gd2O3

distance à l'extrémité de la fibre (μm)

FIGURE 4

FIGURE 5

Yb$^{3+}$(0-18%): Gd$_2$O$_3$
lifetime at 1030nm
$\lambda$ exc=979nm

FIGURE 6

FIGURE 7

FIGURE 8

FIGURE 9

FIGURE 10

FIGURE 11

Office européen
des brevets

**RAPPORT DE RECHERCHE EUROPEENNE**

Numéro de la demande

EP 00 11 1371

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 096 663 A (TATARCHUK BRUCE J) 17 mars 1992 (1992-03-17) * revendication 1 * --- | 1-17 | G01N1/28 G01M11/00 G01N33/00 |
| A,D | DANIELSON E ET AL: "A COMBINATORIAL APPROACH TO THE DISCOVERY AND OPTIMIZATION OF LUMINESCENT MATERIALS" NATURE,GB,MACMILLAN JOURNALS LTD. LONDON, vol. 389, no. 6654, 30 octobre 1997 (1997-10-30), pages 944-948, XP000867785 ISSN: 0028-0836 * abrégé * --- | 1-17 | |
| A | LIN C -Y ET AL: "EXTRUSION PROCESS FOR MANUFACTURE OF BULK FUNCTIONALLY GRADED MATERIALS" POWDER METALLURGY,GB,METALS SOCIETY. LONDON, vol. 39, no. 3, 1996, pages 219-222, XP000641583 ISSN: 0032-5899 * page 220, colonne 2, alinéa 2 - page 221, colonne 1, alinéa 1 * --- | 1 | |
| A | US 5 167 271 A (LANGE FREDERICK F ET AL) 1 décembre 1992 (1992-12-01) * revendications 15,16 * --- | 1-17 | |
| A | US 4 217 399 A (DOBO EMERICK J) 12 août 1980 (1980-08-12) * revendication 1 * --- | 1 | |

**DOMAINES TECHNIQUES RECHERCHES (Int.Cl.7)**

G01N
G01M

-/--

Le présent rapport a été établi pour toutes les revendications

| Lieu de la recherche | Date d'achèvement de la recherche | Examinateur |
|---|---|---|
| MUNICH | 27 novembre 2000 | Thomte, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
............................................................
& : membre de la même famille, document correspondant

EPO FORM 1503 03.82 (P04C02)

Office européen
des brevets

**RAPPORT DE RECHERCHE EUROPEENNE**

Numéro de la demande

EP 00 11 1371

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | JONES S P ET AL: "Structural development in mesophase pitch based carbon fibers produced from naphthalene" , CARBON,US,ELSEVIER SCIENCE PUBLISHING, NEW YORK, NY, VOL. 35, NR. 10-11, PAGE(S) 1533-1543 XP004098174 ISSN: 0008-6223 * abrégé * | 1-17 | |
| A | US 3 864 626 A (MACLEAN ALEXANDER F ET AL) 4 février 1975 (1975-02-04) * colonne 1, ligne 29 – colonne 1, ligne 65 * * colonne 7, ligne 65 – colonne 8, ligne 36 * | 1 | |

DOMAINES TECHNIQUES RECHERCHES (Int.Cl.7)

Le présent rapport a été établi pour toutes les revendications

| Lieu de la recherche | Date d'achèvement de la recherche | Examinateur |
|---|---|---|
| MUNICH | 27 novembre 2000 | Thomte, M |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 00 11 1371

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-11-2000

| Document brevet cité au rapport de recherche | | Date de publication | Membre(s) de la famille de brevet(s) | | Date de publication |
|---|---|---|---|---|---|
| US 5096663 | A | 17-03-1992 | AU | 8218491 A | 31-12-1991 |
| | | | WO | 9118698 A | 12-12-1991 |
| US 5167271 | A | 01-12-1992 | AUCUN | | |
| US 4217399 | A | 12-08-1980 | US | 4089921 A | 16-05-1978 |
| | | | US | 4287254 A | 01-09-1981 |
| US 3864626 | A | 04-02-1975 | US | 3979666 A | 07-09-1976 |

EPO FORM P0460

Pour tout renseignement concernant cette annexe : voir Journal Officiel de l'Office européen des brevets, No.12/82